# EUROPÄISCHE PATENTSCHRIFT

(11) **EP 2 051 756 B1**
(45) Veröffentlichungstag und Bekanntmachung des Hinweises auf die Patenterteilung: **09.03.2011**
(21) Anmeldenummer: 07817417.4
(22) Anmeldetag: 08.08.2007
(51) Int. Cl.: A61M 11/04, A61M 15/00, A24F 47/00, A61F 7/03

(54) **VERDAMPFUNGSELEMENT FÜR FLÜSSIGKEITEN**
EVAPORATION ELEMENT FOR LIQUIDS
ÉLÉMENT D'ÉVAPORATION POUR LIQUIDES

(30) Priorität: 08.08.2006 DE 102006037031
(43) Veröffentlichungstag der Anmeldung: 29.04.2009
(73) Patentinhaber: Stirzel, Alexander, 89075 Ulm (DE)
(72) Erfinder: Stirzel, Alexander, 89075 Ulm (DE)
(74) Vertreter: Esslinger, Alexander
(86) Internationale Anmeldenummer: PCT/DE2007/001411
(87) Internationale Veröffentlichungsnummer: WO 2008/017298

(56) Entgegenhaltungen:
- WO-A-2005/020726
- FR-A- 2 065 782
- FR-A- 2 818 152
- GB-A- 644 025
- US-A1- 2001 042 546
- US-A1- 2005 236 006

## Beschreibung

### Gebiet der Erfindung

Die Erfindung betrifft ein Verdampfungselement für Flüssigkeiten, insbesondere wässrige Lösungen, die Aromen sowie beispielsweise Koffein, Nikotin und andere enthalten können, vorzugsweise zur Inhalation der verdampften Flüssigkeit.

### Verwandter Stand der Technik

Vorrichtungen zur Erhitzung von Flüssigkeiten, die Aromastoffe, wie etwa ätherische Öle oder dergleichen enthalten, zum Zwecke der Inhalation des mit den Aromastoffen getränkten Flüssigkeitsdampfes sind seit langem bekannt.

Medizinische Anwendungen der Inhalation von verdampfenden Flüssigkeiten liegen beispielsweise in der Inhalation von ätherischen Ölen zur Linderung von Erkältungsbeschwerden und Atemwegserkrankungen. Eine weitere Anwendung liegt in der Nikotinverabreichung, wie in dem Patent DE 10 2004 033 579 B4 beschrieben ist. Dabei wird ein Gemisch aus Wasser, Nikotin und Aromastoffen verdampft, welche Aromastoffe schon bei relativ geringen Temperaturen zwischen 50 und 90°C verdampfen. Nikotin selbst verdampft erst bei ca. 300°C, ist jedoch hygroskopisch und wasserdampfdestillierbar. Deshalb besteht die Möglichkeit, ein homogenes Nikotin-Wasser-Gemisch schon bei verhältnismäßig geringen Temperaturen zu verdampfen. Durch die Aufnahme des Nikotin-Aroma-Dampfgemisches ohne gleichzeitige Aufnahme kanzerogener Substanzen (hauptsächlich Kondensat) wie bei Nikotinaufnahme durch Rauch eröffnet diese Form der Nikotinaufnahme die Möglichkeit zum Nikotingenuß, eine wohldosierte Nikotinmenge aufzunehmen, ohne gleichzeitig krebserzeugende Stoffe aufnehmen zu müssen und ohne umstehende Personen oder dgl. zu beeinträchtigen.

Aus der DE 10 2004 033 579 B4 ist es bekannt, das fein poröse Keramikmaterial mit der verdampfenden Lösung zu tränken und den Dampf für die Inhalation durch Erhitzung des Keramikelements wieder freizusetzen. Als Wärmequelle für die Erhitzung kann dabei eine elektrische Wärmequelle oder eine externe Wärmequelle wie ein Feuerzeug oder dergleichen eingesetzt werden. Für eine elektrische Wärmequelle ist jedoch ein großer Energiebedarf erforderlich, der nur durch eine vergleichsweise schwere und voluminöse Batterie befriedigt werden kann. Insbesondere für ein portables Inhaliergerät ist eine derartige Batterie jedoch unpraktisch, zu schwer und zu teuer.

Externe Wärmequellen wie Feuerzeuge oder dergleichen können aufgrund der sehr hohen Flammentemperatur das Inhaliergerät beschädigen und verrußen und sind daher auch wenig benutzerfreundlich. Desweiteren ist das Hantieren mit offener Flamme in vielerlei Hinsicht mit Gefahren verbunden und aufgrund entstehender Verbrennungsprodukte unerwünscht.

Aus der EP 0 371 282 A ist eine Aerosol-Verdampfungseinrichtung bekannt aufweisend eine nicht auf einem Verbrennungsvorgang beruhende Wärmequelle beispielsweise enthaltend Kalziumoxid, das bei Berührung mit Wasser Wärme erzeugt sowie ein von der Wärmequelle separiertes, aber in thermischem Kontakt mit dieser befindliches Reservoir für das zu erhitzende Aerosol.

Die GB 644 025 A beschreibt eine Verdampfungseinrichtung für Flüssigkeiten, insbesondere wässrige Lösungen in Ampullenform mit einem Glasreservoir, zur Speicherung der zu verdampfenden Flüssigkeit, und einem von dem ersten Glasreservoir getrennten zweiten Reservoir mit einem Reaktionsmittel, welches in Berührung mit der der zu verdampfenden Flüssigkeit eine exotherme chemische Reaktion ausführt. Drückt der Benutzer auf das ampullenförmige Gebilde, bricht das Glasreservoir, so dass das Reaktionsmittel in Kontakt mit der zu verdampfenden Flüssigkeit kommt und die Flüssigkeit über eine öffnung ungerichtet verdampft und entweicht.

Die WO 2005/020726 A1 beschreibt eine geschlossene Raucheinrichtung als Zigarettenersatz, in der eine Tabakmischung oder ähnliches kontrolliert verbrannt wird.

### Zusammenfassung der Erfindung

Der vorliegenden Erfindung liegt daher die Aufgabe zu Grunde, ein Verdampfungselement für Flüssigkeiten, zu schaffen, dass kompakt und preiswert ist und vielseitig insbesondere zu Inhalationszwecken einsetzbar ist.

Zur Lösung der Aufgabe wird ein Verdampfungselement für Flüssigkeiten

Bei dem erfindungsgemäßen Verdampfungselement wird die zu verdampfende Flüssigkeit durch in Kontakt bringen der Flüssigkeit selbst mit dem Reaktionsmittel, beispielsweise Kalziumoxid, erhitzt. Durch Betätigung des Öffnungselements wird die Folie, die die zu verdampfende Flüssigkeit von dem Reaktionsmittel trennt, perforiert, so dass die Flüssigkeit mit dem Reaktionsmittel reagiert und Wärme frei wird, die die Flüssigkeit zum Verdampfen bringt. Gegenüber der aus der EP 0 371 282 A bekannten Aerosol-Verdampfungseinrichtung liegt der durch die Erfindung erreichte Vorteil insbesondere in der einfacheren, kostengünstigeren und platzsparenderen Konstruktion. Mit dem beim erfindungsgemäß vorliegenden Verdampfungselement wird die platzsparendste und günstigste Möglichkeit, auf kleinem Raum Energie portabel bereitzustellen, genutzt, die Speicherung und Freisetzung auf chemischen Wege.

Das erfindungsgemäße Verdampfungselement weist ein über ein Scharnier schwenkbar an dem Gehäuse angebrachtes Deckelteil auf sowie vorzugsweise ein wärme- und wasserfestes Gehäuse etwa aus Kunststoff, z.B. Polysulfon oder Polycarbonat oder dgl. .

Das Öffnungselement ist durch an der Unterseite des schwenkbar an dem Gehäuse angebrachten Deckelteils angeordnete Dorne oder Stifte gebildet. Alternative Öffnungsmechanismen etwa basierend auf einem ähnlich einer Taucheruhr angebrachten Drehring sind jedoch im Rahmen der Erfindung ebenso möglich wie etwa ein seitlich angeordneter Dorn im Gehäuse oder am Verdampfungselement selbst.

Das zweite Reservoir kann als Reaktionsmittel Calciumoxid enthalten. Eine Berührung mit der zu verdampfenden wäßrigen Lösung führt zu einer exothermen chemischen Reaktion.

Die zu verdampfende Flüssigkeit kann eine wässrige Lösung enthaltend Koffein, Nikotin und/oder andere Aromastoffe sein.

Das Verdampfungselement ist vorzugsweise in ein wärme- und wasserfestes Gehäuse bestehend beispielsweise aus Metall, Ton oder vorzugsweise Kunststoff, oder dergleichen eingebettet. Das Gehäuse weist eine Öffnung zur Inhalation der verdampfenden Flüssigkeit auf, z.B. in Form eines klappbaren Schnorchels.

Verdampfungselement mit Gehäuse können so klein und handlich ausgebildet sein, dass sich ein tragbares Inhalationsgerät ergibt, das auch z.B. als Armband ähnlich einer Uhr getragen werden kann.

### Kurze Beschreibung der Zeichnungen

Die Erfindung wird im folgenden anhand eines bevorzugten Ausführungsbeispiels unter Bezugnahme auf die beiliegenden Zeichnungen beschrieben.
Figur 1 zeigt ein Ausführungsbeispiel des erfindungsgemäßen Verdampfungselements ohne Gehäuse in Querschnittsdarstellung.
Figur 2 zeigt das Verdampfungselement mit Gehäuse und geschlossenem Deckel in Querschnittsdarstellung.
Figur 3 zeigt das Verdampfungselement mit Gehäuse und geöffnetem Deckel in Querschnittsdarstellung..

### Detaillierte Beschreibung der Erfindung

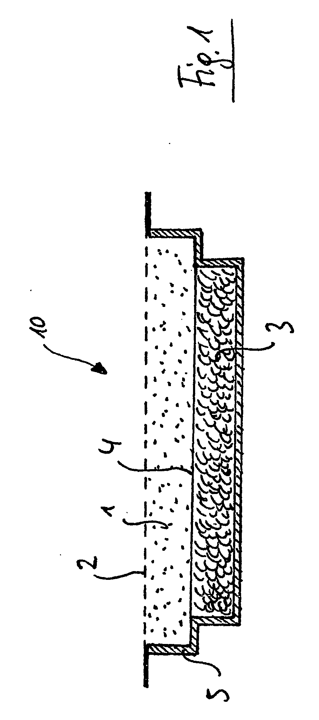

Figur 1 zeigt in Querschnittsdarstellung ein Ausführungsbeispiel des erfindungsgemäßen Verdampfungselements ohne Gehäuse, das insgesamt mit Bezugsziffer 10 bezeichnet ist. Das Verdampfungselement besteht aus einem ersten, oberen Reservoir 1, derals Flüssigkeitspeicher für die zu verdampfende wässrige Lösung dient. Die wässrige Lösung kann dabei Koffein, Nikotin und/oder andere Aromastoffe enthalten.

Das erste Reservoir 1 ist durch eine dünne Kunststoffolie 4 von einem zweiten Reservoir 3 getrennt, das ein Reaktionsmittel wie etwa Calziumoxid enthält. Das erste Reservoir 1 und das zweite Reservoir 3 sind in einer vorzugsweise wasser-und wärmefesten Wanne 5 oder einem Beutel, bevorzugt aus hitzebeständiger Kunststoffolie, untergebracht.

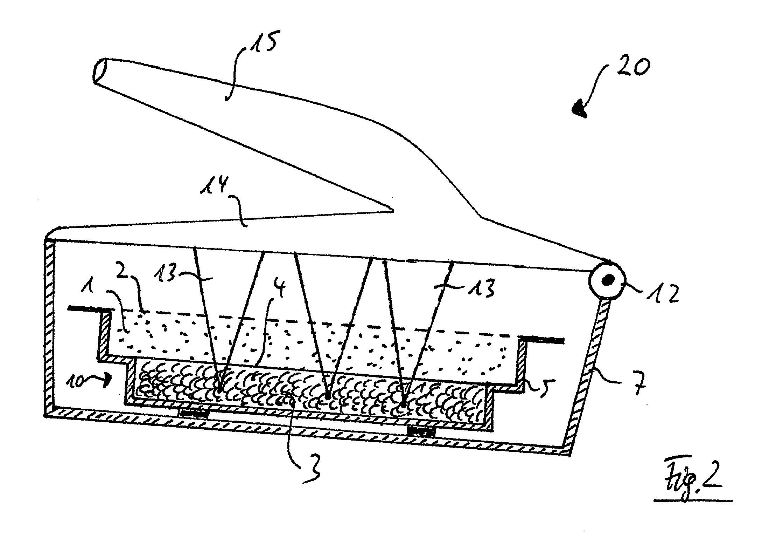

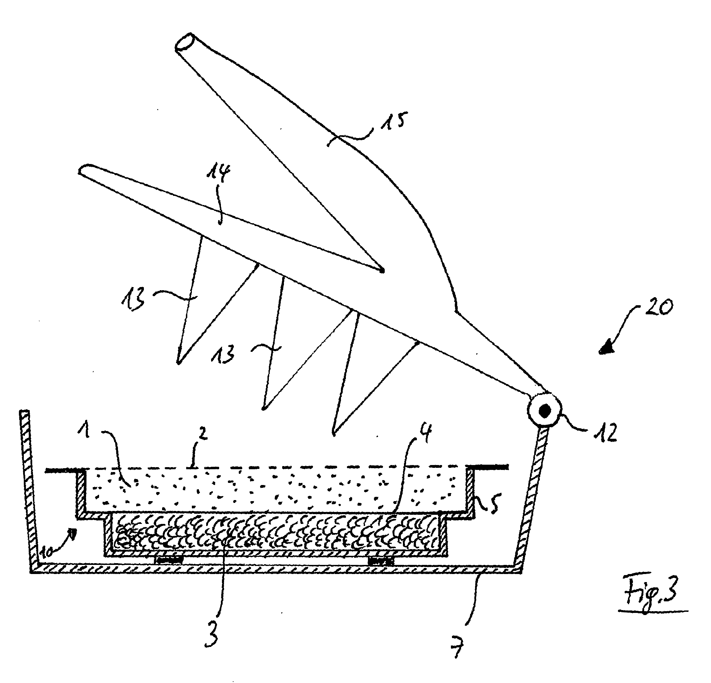

Kommt die in dem ersten Reservoir 1 enthaltene, zu verdampfende Flüssigkeit in Kontakt mit dem im zweiten Reservoir 3 befindlichen Reaktionsmittel, so erzeugt die resultierende chemische Reaktion genügend Wärme, um die Flüssigkeit mit den enthaltenen Aromastoffen zu verdampfen oder zu verdunsten. Um diese Reaktion auszulösen, muß die Schutzfolie 4 perforiert werden, wozu Dorne oder Stifte 13 dienen können, die in dem in den Figuren 2 und 3 gezeigten Ausführungsbeispiel an der Innenseite des schwenkbaren Deckels 14 des Gehäuses angeordnet sind. Andere Mechanismen zur Perforation der Folie 4 sind jedoch im Rahmen der Erfindung selbstverständlich ebenso möglich.

Die Figuren 2 und 3 zeigen das insgesamt mit 20 bezeichnete Verdampfungselement mit Gehäuse und mittels Scharnier 12 schwenkbar angebrachtem Deckel 14 in Querschnittsdarstellung, wobei der Deckel 14 in der in Figur 2 gezeigten Ansicht geöffnet und in der in Figur 3 gezeigten Ansicht geschlossen ist.

Der Benutzer startet den Verdampfungsvorgang durch Schließen des Deckels 14, wodurch die Folie 4 durch die Dorne 13 perforiert und die chemische Reaktion gestartet wird. Die verdampfende, die Aromastoffe enthaltende Flüssigkeit entweicht über eine zweite Folie 2 aus vorzugsweise bestehend aus einer mit Öffnungen versehenen Kunststoffolie. Alternativ kann diese zweite Folie 2 jedoch auch weggelassen werden. Der aufsteigende Dampf gelangt dann duch die Öffnung 15 im Deckel 14 nach draussen.

Zur Wärmeisolierung nach außen weist das erfindungsgemäße Verdampfungselement vorzugsweise ein wärmeisolierendes Gehäuse 7 aus Kunststoff, Keramik oder einem anderen geeigneten Material auf. Das in Figuren 2 und 3 gezeigte Ausführungsbeispiel zeigt ein Gehäuse 7 mit einem Deckel 14, der eine zur Inhalation geeignete Öffnung 15 aufweist, die mit einem Schlauch oder dergleichen versehen werden kann. Anders geformte Gehäuseformen sind jedoch ebenfalls möglich. Der Gestaltungsfreiheit sind hier kaum Grenzen gesetzt. Auch das Verdampfungselement selbst kann anstatt wie in den Figuren 1 - 3 gezeigt rechteckig oder quadratisch auch kreisförmig oder oval oder dergleichen gestaltet sein.

Die Erfindung liefert so ein kompaktes und preiswertes Verdampfungselement für Flüssigkeiten mit einer chemischen Wärmequelle, die nur einen geringen Raum- und Gewichtsbedarf hat. Dadurch, dass die zu verdampfende Flüssigkeit selbst mit dem Reaktionsmittel reagiert, entfällt das in der aus der EP 0 371 282 A bekannten Aerosol-Verdampfungseinrichtung notwendige zusätzliche Wasserreservoir. Dadurch verringern sich Gewicht, Aussenmaße und Kosten in erheblichem Umfang. Das Verdampfungselement ist so auch für ein mobiles Inhalationsgerät geeignet und kann nach Gebrauch leicht ausgetauscht werden, wodurch eine bequeme und hygienische Handhabung ermöglicht wird.

## Patentansprüche

1. Verdampfungselement für Flüssigkeiten, insbesondere wässrige Lösungen, aufweisend:
ein erstes Reservoir (1) zur Speicherung der zu verdampfenden Flüssigkeit,
ein durch eine Folie (4) von dem ersten Reservoir (1) getrenntes zweites Reservoir (3) mit einem Reaktionsmittel, welches in Berührung mit der zu verdampfenden Flüssigkeit eine exotherme chemische Reaktion ausführt, und
ein Öffnungselement (13), das bei Betätigung die Folie (4) perforiert und so das Reaktionsmittel in Kontakt mit der zu verdampfenden Flüssigkeit bringt-**gekennzeichnet durch** ein mit einem Scharnier (12) schwenkbar an dem Gehäuse angebrachtes Deckelteil (14), wobei das Öffnungselement **durch** an der Unterseite des Deckelteils (14) angeordnete Dorne (13) oder Stifte (13) gebildet ist, und wobei das Deckelteil (14) ferner mit einer Öffnung (15) zum Entweichen der verdampfenden Flüssigkeit versehen ist.

2. Verdampfungselement nach Anspruch 1, ferner aufweisend ein wärme- und wasserfestes Gehäuse (7), vorzugsweise aus hitzebeständigem Kunststoff, Polycarbonat oder dgl.

3. Verdampfungselement nach einem der Ansprüche 1 oder 2, wobei die Folie (4) eine dünne Kunststoffolie ist.

4. Verdampfungselement nach einem der Ansprüche 1 - 3, wobei das zweite Reservoir (3) ein exotherm reaktives Stoffgemisch enthält.

5. Verdampfungselement nach Anspruch 4, wobei das exotherm reaktive Stoffgemisch einen hohen Gehalt an Calciumoxid enthält.

6. Verdampfungselement nach einem der Ansprüche 1 - 5, wobei die zu verdampfende Flüssigkeit eines wässrige Lösung enthaltend Koffein, Nikotin und/oder andere Aromastoffe ist.

7. Verdampfungselement nach einem der Ansprüche 1 - 6, ferner aufweisend eine zweite Folie (2) zur Abdeckung der ersten Malerialschicht (1).

8. Verdampfungselement nach Anspruch 7, wobei die zweite Folie (2) aus Kunststoffmaterial besteht.

9. Verdampfungselement nach einem der Ansprüche 1 - 8, aufweisend ein beispielsweise am Handgelenk tragbares Gehäuse (7).

## Claims

1. Vaporization element for liquids, in particular aqueous solution, comprising:
a first reservoir (1) for storage of the liquid to be vaporized,
a second reservoir (3), separated from the first reservoir (1) by a film (4), with a reagent which carries out an exothermic chemical reaction in contact with the liquid to be vaporized, and
an opening element (13) which, when activated, perforates the film (4) and in this way brings the reagent into contact with the liquid to be vaporized,
**characterized by** a cover part (14) mounted on the housing with a hinge (12) such that it pivots, wherein the opening element is formed by mandrels (13) or pins (13) arranged on the under-side of the cover part (14), and wherein the cover part (14)is provided with an opening (15) allowing escape of the vaporizing liquid.

2. Vaporization element according to claim 1, furthermore comprising a heat-resistant and waterproof housing (7), preferably of heat-resistant plastic, polycarbonate or the like.

3. Vaporization element according to one of claims 1 or 2, wherein the film (4) is a thin film of plastic.

4. Vaporization element according to one of claims 1 - 3, wherein the second reservoir (3) contains an exothermically reactive substance mixture.

5. Vaporization element according to claim 6, wherein the exothermically reactive substance mixture contains a high content of calcium oxide.

6. Vaporization element according to one of claims 1 - 5, wherein the liquid to be vaporized is an aqueous solution containing caffeine, nicotine and/or other aroma substances.

7. Vaporization element according to one of claims 1 - 6, furthermore comprising a second film (2) for covering the first layer of material (1).

8. Vaporization element according to claim 7, wherein the second film (2) is made of a plastic material.

9. Vaporization element according to one of claims 1 - 8, comprising a housing (7) which, for example, can be worn on the wrist.

## Revendications

1. Elément d'évaporation pour des liquides, en particulier des solutions aqueuses, présentant
un premier réservoir (1) pour le stockage du liquide à évaporer,
un second réservoir (3) séparé par un film (4) du premier réservoir (1) avec un agent de réaction, qui met en oeuvre une réaction chimique exothermique en contact avec le liquide à évaporer, et
un élément d'ouverture (13), qui perfore le film (4) en cas d'actionnement et amène ainsi l'agent de réaction en contact avec le liquide à évaporer, **caractérisé par** une partie de couvercle (14) placée sur le boîtier de façon à pouvoir basculer avec une charnière (12), l'élément d'ouverture étant formé par des mandrins (13) ou des goupilles (13) disposés sur le côté inférieur de la partie de couvercle (14) et la partie de couvercle (14) étant dotée également d'une ouverture (15) pour l'échappement du liquide qui s'évapore.

2. Elément d'évaporation selon la revendication 1, présentant également un boîtier (7) résistant à la chaleur et à l'eau, de préférence à base de matière plastique résistante à la chaleur, de polycarbonate ou similaire.

3. Elément d'évaporation selon l'une quelconque des revendications 1 ou 2, le film (4) étant un film plastique mince.

4. Elément d'évaporation selon l'une quelconque des revendications 1 à 3, le second réservoir (3) contenant un mélange de produit réactif de façon exothermique.

5. Elément d'évaporation selon la revendication 4, le mélange de produit réactif de façon exothermique contenant une teneur élevée en oxyde de calcium.

6. Elément d'évaporation selon l'une quelconque des revendications 1 à 5, le liquide à évaporer étant une solution aqueuse contenant de la caféine, de la nicotine, et/ou d'autres produits aromatiques.

7. Elément d'évaporation selon l'une quelconque des revendications 1 à 6, présentant également un second film (2) pour le recouvrement de la première couche de matériau (1).

8. Elément d'évaporation selon la revendication 7, le second film (2) se composant de matière plastique.

9. Elément d'évaporation selon l'une quelconque des revendications 1 à 8, présentant un boîtier (7) pouvant être porté par exemple au poignet.
